(19) Europäisches Patentamt
European Patent Office
Office européen des brevets

(11) Veröffentlichungsnummer: **0 196 038 B1**

(12) # EUROPÄISCHE PATENTSCHRIFT

(45) Veröffentlichungstag der Patentschrift: **02.01.92**

(51) Int. Cl.[5]: **C07D 405/06**, A01N 43/50, A01N 43/653

(21) Anmeldenummer: **86103969.1**

(22) Anmeldetag: **22.03.86**

Die Akte enthält technische Angaben, die nach dem Eingang der Anmeldung eingereicht wurden und die nicht in dieser Patentschrift enthalten sind.

(54) **Azolylmethyloxirane - ihre Herstellung und Verwendung als Pflanzenschutzmittel.**

(30) Priorität: **29.03.85 DE 3511411**
**12.10.85 DE 3536529**

(43) Veröffentlichungstag der Anmeldung:
**01.10.86 Patentblatt 86/40**

(45) Bekanntmachung des Hinweises auf die Patenterteilung:
**02.01.92 Patentblatt 92/01**

(84) Benannte Vertragsstaaten:
**AT BE CH DE FR GB LI**

(56) Entgegenhaltungen:
**EP-A- 0 094 564**

(73) Patentinhaber: **BASF Aktiengesellschaft**
**Carl-Bosch-Strasse 38**
**W-6700 Ludwigshafen(DE)**

(72) Erfinder: **Karbach, Stefan, Dr.**
**Sperlinggasse 3**
**W-6700 Ludwigshafen(DE)**
Erfinder: **Janssen, Bernd, Dr.**
**Leuschnerstrasse 18 a**
**W-6700 Ludwigshafen(DE)**
Erfinder: **Meyer, Norbert, Dr.**
**Dossenheimer Weg 22**
**W-6802 Ladenburg(DE)**
Erfinder: **Sauter, Hubert, Dr.**
**Neckarpromenade 20**
**W-6800 Mannheim 1(DE)**
Erfinder: **Ammermann, Eberhard, Dr.**
**Sachsenstrasse 3**
**W-6700 Ludwigshafen(DE)**
Erfinder: **Pommer, Ernst-Heinrich, Dr.**
**Berliner Platz 7**
**W-6703 Limburgerhof(DE)**

**EP 0 196 038 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Azolverbindungen, Verfahren zu ihrer Herstellung und diese enthaltende Fungizide.

Aus der europäischen Patentveröffentlichung 94 564 sind Azolverbindungen, insbesondere das 2-(1,2,4-Triazol-1-yl-methyl)-2-(4-chlorphenyl)-3-(2,4-dichlorphenyl)-oxiran bekannt, deren Wirkung als Fungizide nicht in allen Fällen befriedigt.

Es wurde nun gefunden, daß Verbindungen der Formel I

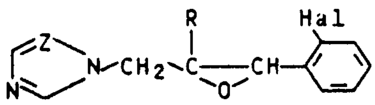

I

in der R 4-Phenylsulfonylphenyl, Naphthyl, Biphenyl oder Phenyl bedeutet, wobei der Phenylrest durch Halogen, Nitro, Phenoxy, Alkyl, Alkoxy oder Halogenalkyl mit jeweils 1 bis 4 Kohlenstoffatomen substituiert sein kann,
in der Hal Fluor, Chlor oder Brom
und in der = Z- : = CH- oder = N- bedeutet,
sowie deren für Pflanzen verträgliche Säureadditions- und Metallsalze eine bessere fungizide Wirkung, insbesondere gegen Getreidekrankheiten, besitzen als die bekannten Azolverbindungen.

Die Verbindungen der Formel I enthalten chirale Zentren und werden im allgemeinen in Form von Racematen bzw. als Diastereomerengemische von erythro- sowie threo-Formen erhalten. Die erythro- und threo-Diastereomeren lassen sich bei den erfindungsgemäßen Verbindungen in üblicher Weise, beispielsweise aufgrund ihrer unterschiedlichen Löslichkeit oder durch Säulenchromatographie trennen und in reiner Form isolieren. Aus solchen einheitlichen Diastereomerenpaaren kann man mit bekannten Methoden einheitliche Enantiomere erhalten. Als fungizide Mittel kann man sowohl die einheitlichen Diastereomere bzw. Enantiomere wie auch deren bei der Synthese anfallende Gemische verwenden.

R bedeutet beispielsweise: 1-Naphthyl, 2-Naphthyl, p-Biphenyl, Phenyl, 2-Chlorphenyl, 3-Chlorphenyl, 4-Chlorphenyl, 4-Fluorphenyl, 4-Bromphenyl, 2,4-Dichlorphenyl, 3,4-Dichlorphenyl, 3,5-Dichlorphenyl, 3-Chlor-4-methylphenyl, 2-Methoxyphenyl, 3-Methoxyphenyl, 2,4-Dimethoxyphenyl, 3,4-Dimethoxyphenyl, 4-Methoxyphenyl, 4-Ethoxyphenyl, 4-tert.-Butoxyphenyl, 4-Methylphenyl, 4-Ethylphenyl, 4-Isopropylphenyl, 4-tert.-Butylphenyl, 4-Phenoxyphenyl, 3-Phenoxyphenyl, 3-Nitrophenyl, 4-Nitrophenyl, 3-Trifluormethylphenyl, 4-Trifluormethylphenyl und 4-Phenylsulfonylphenyl.

Säureadditionssalze sind beispielsweise die Hydrochloride, Bromide, Sulfate, Nitrate, Phosphate, Oxalate oder Dodecyl-benzolsulfonate. Die Wirksamkeit der Salze geht auf das Kation zurück, so daß es auf das Anion i.a. nicht ankommt. Die erfindungsgemäßen Wirkstoffsalze werden hergestellt durch Umsetzung der Azolylmethyloxirane (I) mit geeigneten Säuren.

Metallkomplexe der Wirkstoffe I oder ihrer Salze können z.B. mit Kupfer, Zink, Zinn, Mangan, Eisen, Kobalt oder Nickel gebildet werden, indem man die Azolylmethyloxirane mit entsprechenden Metallsalzen umsetzt.

Die Verbindungen der Formel I können z.B. hergestellt werden, indem man

a) ein entsprechendes Vorprodukt der Formel II

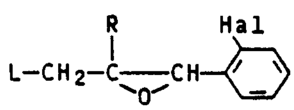

(II),

in welcher L eine nukleophil substituierbare Abgangsgruppe darstellt, mit einem entsprechenden Azol der Formel III

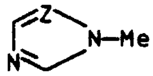

(III),

in der Me ein Wasserstoffatom oder ein Metallatom bedeutet zur Umsetzung bringt.

b) Ein weiterer Weg zur Herstellung einer Verbindung I besteht darin, daß man eine Verbindung der Formel II, in der R und Hal die oben angegebenen Bedeutungen haben und L für eine Hydroxygruppe steht, mit einer Verbindung der Formel IV

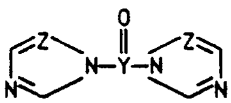

(IV),

in welcher Z die angegebene Bedeutung hat und Y ein Kohlenstoff- oder ein Schwefelatom bedeutet, zur Reaktion bringt oder

c) eine Verbindung der Formel V

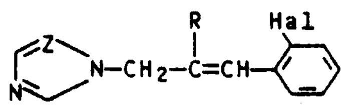

(V),

in welcher Z, R und Hal die angegebene Bedeutung haben, epoxidiert oder

d) eine Verbindung der Formel VI

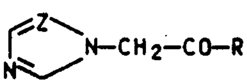

(VI),

in welcher Z und R die angegebene Bedeutung haben, mit einer Verbindung der allgemeinen Formel VII

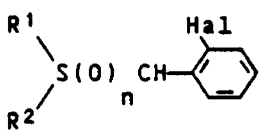

(VII),

in welcher Hal die angegebene Bedeutung hat, $R^1$ sowie $R^2$, die gleich oder verschieden voneinander sein können, eine Methyl- oder Phenyl-gruppe bedeuten und n Null oder Eins bedeutet, zur Reaktion bringt und die so erhaltenen Verbindungen gegebenenfalls in ihre Salze mit physiologisch verträglichen Säuren überführt.

Die Reaktion a) erfolgt - falls Me ein Wasserstoffatom bedeutet -gegebenenfalls in Gegenwart eines Lösungs- oder Verdünnungsmittels, gegebenenfalls unter Zusatz einer anorganischen oder organischen Base und gegebenenfalls unter Zusatz eines Reaktionsbeschleunigers bei Temperaturen zwischen 10 und 120 °C. Zu den bevorzugten Lösungs- oder Verdünnungsmitteln gehören Ketone wie Aceton, Methylethylketon oder Cyclohexanon, Nitrile wie Acetonitril, Ester wie Essigsäureethylester, Ether wie Diethylether, Tetrahydrofuran oder Dioxan, Sulfoxide wie Dimethylsulfoxid, Amide wie Dimethylformamid, Dimethylacetamid oder N-Methylpyrrolidon, ferner Sulfolan oder entsprechende Gemische.

Geeignete Basen, dei gegebenenfalls auch als säurebindende Mittel bei der Reaktion verwendet werden können, sind beispielsweise Alkalihydroxid wie Lithium-, Natrium- oder Kaliumhydroxid; Alkalicarbonate wie Natrium- oder Kaliumcarbonat oder Natrium- und Kaliumhydrogencarbonat, Überschüsse an 1,2,4-Triazol, Pyridin oder 4-Dimethylaminopyridin. Es können aber auch andere übliche Basen verwendet werden.

Als Reaktionsbeschleuniger kommen vorzugsweise Metallhalogenide wie Natriumiodid oder Kaliumiodid, quaternäre Ammoniumsalze wie Tetrabutylammoniumchlorid, -bromid oder -iodid, Benzyl-tri-ethylammoniumchlorid oder -bromid oder Kronenether wie 12-Krone-4, 15-Krone-5, 18-Krone-6, Dibenzo-18-Krone-6 oder Dicyclohexano-18-Krone-6 in Frage.

Die Umsetzung wird im allgemeinen bei Temperaturen zwischen 20 und 150 °C, drucklos oder unter

Druck, kontinuierlich oder diskontinuierlich durchgeführt.

Ist Me ein Metallatom wird die Reaktion a) gegebenenfalls in Gegenwart eines Lösungs- oder Verdünnungsmittels und gegebenenfalls unter Zusatz eine starken anorganischen oder organischen Base bei Temperaturen zwischen -10 und 120 °C durchgeführt. Zu den bevorzugten Lösungs- oder Verdünnungsmitteln gehören Amide wie Dimethylformamid, Diethylformamid, Dimethylacetamid, Diethylacetamid, N-Methylpyrrolidon, Hexamethyl-phosphortriamid, Sulfoxide wie Dimethylsulfoxid und schließlich Sulfolan.

Geeignete Basen, die gegebenenfalls auch als säurebindende Mittel bei der Reaktion verwendet werden können, sind beispielsweise Alkalihydride wie Lithium-, Natrium- und Kaliumhydrid, Alkaliamide wie Natrium- und Kaliumamid, ferner Natrium- oder Kalium-tert.-butoxid, Lithium-, Natrium- oder Kalium-triphenylmethyl und Naphthalinlithium, -natrium oder -kalium.

Für die Reaktion b) kommen als Verdünnungsmittel polare organische Lösungsmittel wie Nitrile, z.B. Acetonitril, Sulfoxide, z.B. Dimethylsulfoxid, Formamide, z.B. Dimethylformamid, Ketone, z.B. Aceton, Ether, z.B. Diethylether, Tetrahydrofuran und insbesondere Chlorkohlenwasserstoffe, z.B. Methylenchlorid und Chloroform, in Frage.

Man arbeitet im allgemeinen, zwischen 0 und 100 °C, vorzugsweise bei 20 bis 80 °C. Bei Anwesenheit eines Lösungsmittels wird zweckmäßigerweise beim Siedepunkt des jeweiligen Lösungsmittels gearbeitet.

Bei der Durchführung des Verfahrens b) setzt man auf 1 Mol der Verbindung der Formel II (L = OH), vorzugsweise etwa 1 Mol Carbonyl-bis-1,2,4 triazol-(1) bzw. Carbonyl-bis-imidazol-(1) oder 1 Mol Sulfonyl-1,2,4-triazol-(1) bzw. Sulfonyl-bis-imidazol-(1) ein oder erzeugt Sulfonyl-bis-1,2,4-triazol-(1) bzw. Sulfonyl-bis-imidazol-(1) in situ. Zur Isolierung der Verbindungen der Formel I wird das Lösungsmittel abdestilliert, der Rückstand mit einem organischen Solvens aufgenommen und mit Wasser gewaschen.

Die neuen Ausgangsverbindungen II erhält man durch Epoxidierung der entsprechenden Olefine IX:

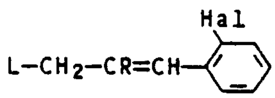

(IX)

(vgl. G. Dittus in Houben-Weyl-Müller, Methoden der organischen Chemie, Georg Thieme Verlag, Stuttgart, 1965, Bd. VI, 3, Seite 385 ff).

Die Verbindung IX stellt man her, indem man Olefine der Formel X

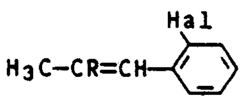

(X)

nach bekannten Methoden in Allylposition halogeniert oder oxidiert.

Geeignete Halogenierungsreagenzien sind N-Chlor -und N-Bromsuccinimid in halogenierten Kohlenwasserstoffen wie Tetrachlorkohlenstoff, Trichlorethan oder Methylenchlorid bei Temperaturen zwischen 20 und 100 °C. Zur Allyloxidation verwendet man Perester wie Perbenzoesäure-tert.-butylester oder Peressigsäure-tert.-butylester in Anwesenheit eines Schwermetallsalzes wie z.B. Kupfer-I-chlorid oder Kupfer-I-bromid. Man arbeitet in inerten Lösungsmitteln bei Temperaturen zwischen 10 und 100 °C.

Die so erhaltenen Allyl-halogenide bzw. -alkohole IX werden anschließend in die entsprechenden Epoxide II (L = Halogen, OH) übergeführt. Dazu oxidiert man die Olefine IX mit Peroxycarbonsäuren wie Perbenzoesäure, 3-Chlorperbenzoesäure, 4-Nitroperbenzoesäure, Monoperphthalsäure, Peressigsäure, Perpropionsäure, Permaleinsäure, Monoperbernsteinsäure, Perpelargonsäure oder Trifluorperessigsäure in indifferenten Lösungsmitteln, vorzugsweise chlorierten Kohlenwasserstoffen, z.B.Methylenchlorid, Chloroform, Tetrachlorkohlenstoff, Dichlorethan, aber gegebenenfalls auch in Essigsäure, Essigester, Aceton oder Dimethylformamid, gegebenenfalls in Gegenwart eines Puffers wie Natriumacetat, Natriumcarbonat Natriumhydrogencarbonat, Dinatriumhydrogenphosphat, Triton B. Man arbeitet zwischen 10 und 100 °C und katalysiert die Reaktion gegebenenfals z.B. mit Iod, Natriumwolframat oder Licht. Zur Oxidation eignen sich auch alkalische Lösungen von Wasserstoffperoxid (ca. 30 %ig) in Methanol, Ethanol, Aceton oder Acetonitril bei 25 bis 30 °C sowie Alkylhydroperoxide, z.B. tert.-Butylhydroperoxid, unter Zusatz eines Katalysators, z.B. Natriumwolframat, Perwolframsäure, Molybdänhexacarbonyl oder Vanadylacetylacetonat. Die genannten Oxidationsmittel lassen sich z.T. in situ erzeugen.

Während die so erhaltenen Epoxihalogenide II (L = Halogen) gemäß Verfahren a) sofort umgesetzt werden können, läßt man die entsprechenden Epoxialkohole II (L = OH) entweder mit Verbindungen der

Formel IV gemäß Verfahren b) reagieren oder überführt dieselben in reaktive Ester, die dann mit den Verbindungen III gemäß Verfahren a) umgesetzt werden.

Die Darstellung der reaktiven Ester, die mit III umgesetzt werden, erfolgt nach allgemein bekannten Methoden (Houben-Weyl-Müller, Methoden der organischen Chemie, Georg Thieme Verlag, Stuttgart, 1955, Band 9, Seiten 388, 663, 671). Solche Ester sind beispielsweise Methansulfonsäureester, Trifluormethansulfonsäureester, 2,2,2-Trifluormethansulfonsäureester, Nonafluorbutansulfonsäurester, 4-Methylbenzolsulfonsäureester, 4-Brombenzolsulfonsäureester, 4-Nitrobenzolsulfonsäureester oder Benzolsulfonsäureester.

Die Verbindungen X lassen sich entsprechend allgemein bekannten Verfahren zur Olefinsynthese (Houben-Weyl-Müller, Methoden der organischen Chemie, Georg Thieme Verlag, Stuttgart, 1972 Bd. V, 1b)-herstellen.

Das Verfahren c) erfolgt analog der Epoxidierung der Verbindungen IX.

Die Ausgangsverbindungen V sind bekannt (DE-OS 25 49 798) und lassen sich nach den dort angegebenen Methoden herstellen.

Für das erfindungsgegemäße Verfahren d) setzt man literaturbekannte Azolylketon (z.B. DE-OS 20 63 857) der Formel VI mit Schwefelderivaten der Formel VII um.

Die Alkylidensulfurane VII ( n = 0) und Oxysulfurane VII (n = 1) werden nach bekannten allgemeinen Methoden (z.B. H.O. House, Modern Synthetic Reactions, 2nd Ed., W. A. Benjamin, Menlo Park 1972, S.712 ff) in situ hergestellt und in inerten Lösungsmitteln, vorzugsweise Ethern wie Diethylether, Tetrahydrofuran oder Mischungen aus beiden oder in Kohlenwasserstoffen wie Pentan, Hexan oder Petrolethern bei Temperaturen zwischen -78 und 30 °C. mit den Azolylketonen VI umgesetzt.

Die so erhaltenen Verbindungen der Formel I werden nach üblichen Methoden isoliert, gegebenenfalls gereinigt und gegebenenfalls mit Säuren zu Salzen umgesetzt.

Die folgenden Beispiele erläutern die Herstellung der Wirkstoffe.

1.Herstellung der Ausgangsstoffe

Beispiel A

In eine Lösung von 194,5 g 2-Chlorbenzyltriphenylphosphoniumchlorid und 800 ml trockenem Methanol werden bei 10 °C 30 g Natriummethylat in 300 ml trockenem Methanol eingetragen und nach einer halben Stunde 60 g Acetophenon zugegeben. Die Reaktionslösung wurde 3 Stunden unter Rückfluß gekocht, dann bei Raumtemperatur das abgeschiedene Salz abfiltriert und das Filtrat im Vakuum eingedampft. Durch Digerieren des Rückstandes mit Petrolether (Kp 50 bis 70 °C) wurde vom Triphenylphosphinoxid abgetrennt und die Lösung im Vakuum eingedampft.

Der Rückstand wurde in einem 1 Tetrachlorkohlenstoff aufgenommen und mit 81,7 g N-Bromsuccinimid und 4 g 2,2'-Azoisobuttersäuredinitril unter Rückfluß gekocht. Nach beendeter Reaktion wurde das Succinimid durch Filtration abgetrennt, das Filtrat im Vakuum eingedampft und der Rückstand aus Methanol umkristallisiert. Man erhielt 65,5 g (43 %) Z-1-(2-Chlorphenyl)-2-phenyl-3-brom-propen-1, Fp = 78 °C.

Beispiel B

30 g Z-1-(2-Chlorphenyl)-2-phenyl-3-brom-propen-1 wurden mit 23 g 3-Chlorperoxybenzoesäure in 500 ml Chloroform unter Rückfluß gekocht. Nach beendeter Reaktion wurde die Chloroform-Phase mit wäßriger Natriumhydrogencarbonat-Lösung und Wasser säurefrei gewaschen, über Natriumsulfat getrocknet und im Vakuum eingedampft. Aus dem Rückstand erhielt man 41,3 g (70, 2 %) 2-Brommethyl-2-phenyl-3-(2-chlorphenyl)-oxiran, das anschließend mit Triazol gemäß dem folgenden Beispiel weiterverarbeitet wurde.

II. Herstelung der Endprodukte

Beispiel 1

23 g 1,2,4-Triazol und 5 g Natriumhydrid (80 %ige Dispersion in Mineralöl) wurden in 150 ml N,N-Dimethylformamid suspendiert und bei Raumtemperatur mit einer Lösung aus 32 g 2-Brommethyl-2-phenyl-3-(2-chlorphenyl)-oxiran in 150 ml N,N-Dimethylformamid versetzt. Nach 8 h wurde die Reaktionslösung auf Wasser gegeben und mit Essigsäureethylester extrahiert. Die organische Phase wurde mit Wasser gewaschen, über Natriumsulfat getrocknet und das Lösungsmittel im Vakuum abgedampft. Man erhielt aus Diisopropylether 24 g Z-2-(1,2,4-Triazol-1-yl-methyl)-2-phenyl-3-(2-chlorphenyl)oxiran mit dem Schmelzpunkt 150 °C (Verbindung Nr.1).

Entsprechend Beispiel 1 können die in der Tabelle aufgeführten Verbindungen hergestellt werden.

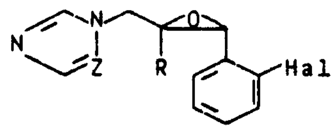

| Nr. | R | Hal | Schmp. | Z | Isomer |
|---|---|---|---|---|---|
| 1 | $C_6H_5$ | Cl | 164-166°C | N | Z |
| 2 | 4-Cl $_6H_4$ | Cl | 166°C | N | Z |
| 3 | 4-Biphenyl | Cl | 191°C | N | Z |
| 4 | 2,4-$Cl_2$-$C_6H_3$ | Cl | | N | Z |
| 5 | 2-Cl-$C_6H_4$ | Cl | | N | Z |
| 6 | 2-F-$C_6H_4$ | Cl | | N | Z |
| 7 | 4-$CH_3$-$C_6H_4$ | Cl | 140°C | N | Z |
| 8 | 4-F-$C_6H_4$ | Cl | 136°C | N | Z |
| 9 | 3-Br-4-F-$C_6H_3$ | Cl | 129-130°C | N | Z |
| 10 | 4-Br-$C_6H_4$ | Cl | | N | Z |
| 11 | 3,4-C $_2$-$C_6H_3$ | Cl | | N | Z |
| 12 | 4-t-$C_4H_9$-$C_6H_4$ | Cl | | N | Z |
| 13 | 3-Cl-$C_6H_4$ | Cl | | N | Z |
| 14 | 3,5-$Cl_2$-$C_6H_3$ | Cl | | N | Z |
| 15 | p-$C_6H_5$-O-$C_6H_4$- | Cl | | N | Z |
| 16 | 4-Cl-$C_6H_4$ | F | 138-140°C | N | Z |
| 17 | $C_6H_5$ | F | 139°C | N | Z |
| 18 | p-Biphenyl | F | | N | Z |
| 19 | 2,4-$Cl_2$-$C_6H_3$ | F | 117°C | N | Z |
| 20 | 2-Cl-$C_6H_4$ | F | | N | Z |
| 21 | 2-F-$C_6H_4$ | F | 128°C | N | Z |
| 22 | 4-$CH_3$-$C_6H_4$ | F | 131°C | N | Z |
| 23 | 4-F-$C_6H_4$ | F | 114°C | N | Z |
| 24 | 3-Br-4-F-$C_6H_3$ | F | 106°C | N | Z |
| 25 | 4-Br-$C_6H_4$ | F | | N | Z |
| 26 | 3,4-$Cl_2$-$C_6H_3$ | F | | N | Z |

| Nr. | R | Hal | Schmp. | Z | Isomer |
|---|---|---|---|---|---|
| 27 | 4-t-$C_4H_9$-$C_6H_4$ | F | | N | Z |
| 28 | 3-Cl-$C_6H_4$ | F | | N | Z |
| 29 | 3,5-$Cl_2$ $C_6H_3$ | F | | N | Z |
| 30 | 2-$C_6H_5$-O-$C_6H_4$ | F | | N | Z |
| 31 | 4-Cl-$C_6H_4$ | Br | | N | Z |
| 32 | $C_6H_5$ | Br | 153°C | N | Z |
| 33 | p-Biphenyl | Br | | N | Z |
| 34 | 2,4-$Cl_2$-$C_6H_3$ | Br | | N | Z |
| 35 | 2-Cl-$C_6H_4$ | Br | | N | Z |
| 36 | 2-F-$C_6H_4$ | Br | | N | Z |
| 37 | 4-$CH_3$-$C_6H_4$ | Br | | N | Z |
| 38 | 4-F-$C_6H_4$ | Br | | N | Z |
| 39 | 3-Br-4-F-$C_6H_4$ | Br | | N | Z |
| 40 | 4-Br-$C_6H_4$ | Br | | N | Z |
| 41 | 3,4-$Cl_2$-$C_6H_3$ | Br | | N | Z |
| 42 | 4-t-$C_4H_9$-$C_6H_4$ | Br | | N | Z |
| 43 | 3-Cl-$C_6H_4$ | Br | | N | Z |
| 44 | 3,5-$Cl_2$-$C_6H_3$ | Br | | N | Z |
| 45 | 2-$C_6H_5$-O-$C_6H_4$ | Br | | N | Z |
| 46 | 4-Cl-$C_6H$ | Cl | 90-92°C | CH | Z/E 70 : 30 |
| 47 | $C_6H_5$ | Cl | 85-87°C | CH | Z/E 15 : 35 |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:

Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Septoria nodorum an Weizen,
Botrytis cinerea (Grauschimmel)an Erdbeeren, Reben,
Cercospora arachidicola an Erdnüssen,
Pyrenophora teres an Gerste,
Pseudocercosporella herpotrichoides an Weizen, Gerste,
Pyricularia oryzae an Reis,

Hemileia vastratrix an Kaffee,
Alternaria solani an Kartoffeln, Tomaten,
Sclerotium rolfsii an Erdnüssen und Rasen-Arten,
Fusarium- und Verticillium-Arten an verschiedenen Pflanzen.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0.02 und 3 kg Wirkstoff oder mehr je ha. Die neuen Verbindungen können auch im Materialschutz eingesetz werden. Bei der Anwendung der Wirkstoffe im Materialschutz u.a. zur Bekämpfung holzzerstörender Pilze wie Coniophora puteana und Polystictus versicolor eingesetzt werden. Die neuen Wirkstoffe können auch als fungizid wirksame Bestandteile öliger Holzschutzmittel zum Schutz von Holz gegen holzverfärbende Pilze eingesetzt werden. Die Anwendung erfolgt in der Weise, daß man das Holz mit diesen Mitteln behandelt, beispielsweise tränkt oder anstreicht.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielweise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 2 mit 10 Gew.-Teilen N-Methyl-α-pyrrolidon und erhält eine Lösung, due zur Anwendung in Form kleinster Tropfen geeignet ist.

II. 20 Gew.-Teile der Verbindung Nr. 8 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanol-amid, 5 Gew.-Teilen Calciumsalz der Dodecylbenzolsulfonsäure und 5 Gew.-Teilen des Anlagerungsproduktes und 40 Mol Ethlyenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 17 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 2 werden in einer Mischung gelöst, die aus 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 8 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

V1. 3 Gew.-Teile der Verbindung Nr. 17 werden mit 97 Gew.-Teilen feinteiligem Kaolin innig vermischt. Man erhält auf diese Weise ein Stäubemittel, das 3 Gew.% des Wirkstoffs enthält.

V11. 30 Gew.-Teile der Verbindung Nr. 1 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter

Haftfähigkeit.

V111. 40 Gew.-Teile der Verbindung Nr. 2 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX.20 Gew.-Teile der Verbindung Nr. 3 werden mit 2 Gew.-Teilen Calciumsalz der Dodecylbenzolsulfonsäure, 8 Gew.-Teilen Fettalkohol-polyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßien Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Für die folgenden Versuche wurde der bekannte Wirkstoff 2-(1,2,4-Triazolyl-1-yl-methyl)-2-(4-chlorphenyl)-3-(2,4-dichlorphenyl)-oxiran (A) (EP 94 564) verwendet.

Versuch 1

Wirksamkeit gegen Weizenmehltau

Blätter von in Töpfen gewachsenen Weizenkeimlingen der Sorte "Frühgold" werden mit wäßriger Spritzbrühe, die 80% Wirkstoff und 20% Emulgiermittel in der Trockensubstanz enthält, besprüht und 24 Stunden nach dem Antrocknen des Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22 °C und 75 bis 80% relativer Luftfeuchtigkeit aufgestellt. Nach 7 Tagen wird das Ausmaß der Mehltauentwicklung ermittelt.

Das Ergebnis des Versuches zeigt, daß beispielsweise bei der Anwendung als 0,025; 0,006 oder 0,0015 %ige (Gew.%) Wirkstoffbrühe die Verbindungen 2, 8, 16, 21, 23 und 17 eine bessere fungizide Wirkung zeigen (97%) als der bekannte Wirkstoff A (90 %).

Versuch 2

Wirksamkeit gegen Weizenbraunrost

Blätter von in Töpfen gewachsenen Weizensämlingen der Sorte "Frühgold" werden mit Sporen des Braunrostes (Puccinia recondita) bestäubt. Danach werden die Töpfe für 24 Stunden bei 20 bis 22 °C in eine Kammer mit hoher Luftfeuchtigkeit (90 bis 95%) gestellt. Während dieser Zeit keimen die Sporen aus und die Keimschläuche dringen in das Blattgewebe ein. Die infizierten Pflanzen werden anschließend mit wäßrigen Spritzbrühen, die 80% Wirkstoff und 20% Emulgiermittel in der Trockensubstanz enthalten, tropfnaß gespritzt. Nach dem Antrocknen des Spritzbelages werden die Versuchspflanzen im Gewächshaus bei Temperaturen zwischen 20 und 22 °C und 65 bis 70% relativer Luftfeuchte aufgestellt. Nach 8 Tagen wird das Ausmaß der Rostpilzentwicklung auf den Blättern ermittelt.

Das Ergebnis des Versuches zeigt, daß beispielsweise bei der Anwendung als 0,006 oder 0,0015 %ige Wirkstoffbrühe die Verbindungen 1, 2, 3, 7, 8, 9, 16, 23, 24 und 17 eine bessere fungizide Wirkung zeigen (97%) als der bekannte Wirkstoff A (70%).

Versuch 3

Wirksamkeit gegen Pyrenophora teres

Gerstenkeimlinge der Sorte "Asse" werden im Zweiblattstadium mit wäßrigen Suspensionen, die 80% Wirkstoff und 20% Emulgator in der Trockensubstanz enthalten, tropfnaß gespritzt. Nach 24 Stunden werden die Pflanzen mit einer Sporensuspension des Pilzes Pyrenophora teres inoculiert und für 48 Stunden in eine Klimakammer mit hoher Luftfeuchtigkeit bei 18 °C gestellt. Anschließend werden die Pflanzen im Gewächshaus bei 20 bis 22 °C und 70% relativer Luftfeuchtigkeit für weitere 5 Tage kultiviert. Dann wird das Ausmaß der Symptomentwicklung ermittelt.

Das Ergebnis des Versuches zeigt, daß beispielsweise bei der Anwendung als 0.05% %ige Wirkstoffbrühe die Verbindungen 1, 2, 3, 16, 21, 24 und 8 eine gute fungizide Wirkung zeigen (97 %).

**Patentansprüche**

**1.** Azolylmethyloxirane der allgemeinen Formel I

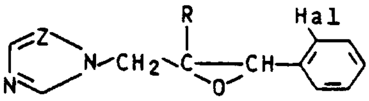

I

in welcher
R 4-Phenylsulfonylphenyl, Naphthyl, Biphenyl oder Phenyl bedeutet, wobei der Phenylrest durch Halogen, Nitro, Phenoxy, Alkyl, Alkoxy oder Halogenalkyl mit jeweils 1 bis 4 Kohlenatomen substituiert sein kann,
Hal Fluor, Chlor oder Brom,
Z CH oder N bedeutet, sowie deren für Pflanzen verträgliche Säureadditions- oder Metallsalze.

**2.** Verfahren zur Herstellung der Azolylmethyloxirane der Formel 1 gemäß Anspruch 1, dadurch gekennzeichnet, daß man

a) eine Verbindung der Formel II

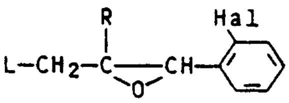

(II),

in welcher R und Hal die angegebenen Bedeutungen haben und L eine nukleophil substituierbare Abgangsgruppe darstellt, mit einer Verbindung der Formel III

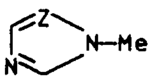

(III),

in der Me ein Wasserstoffatom oder ein Metallatom bedeutet und Z die angegebene Bedeutung hat, zur Umsetzung bringt, oder

b) eine Verbindung der Formel II, in der R und Hal die oben angegebenen Bedeutungen haben und L für eine Hydroxygruppe steht, mit einer Verbindung der Formel IV

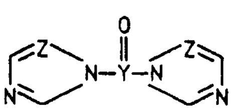

(IV),

in welcher Z die angegebene Bedeutung hat und Y ein Kohlenstoff- oder ein Schwefelatom bedeutet zur Reaktion bringt oder

c) eine Verbindung der Formel V

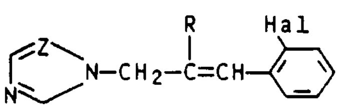

(V),

in welcher Z, R und Hal die angegebene Bedeutung haben, epoxidiert oder

d) eine Verbindung der Formel VI

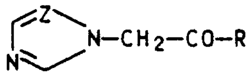 (VI),

in welcher Z und R die angegebene Bedeutung haben, mit einer Verbindung der allgemeinen Formel VII

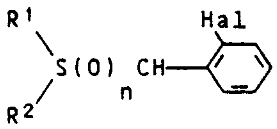 (VII),

in welcher Hal die angegebene Bedeutung hat, $R^1$ sowie $R^2$, die gleich oder verschieden voneinander sein können, eine Methyl- oder Phenyl-Gruppe bedeuten und n Null oder Eins bedeutet, zur Reaktion bringt und die so erhaltenen Verbindungen gegebenenfalls in ihre Salze mit physiologisch bzw. für Pflanzen verträglichen Säuren überführt.

**3.** Fungizides Mittel, enthaltend eine Verbindung der Formel I gemäß Anspruch 1.

**4.** Fungizides Mittel, enthaltend ein Azolylmethyloxiran der allgemeinen Formel I

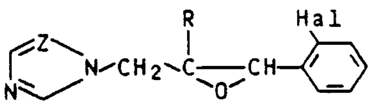 I

in welcher
R 4-Phenylsulfonylphenyl, Naphthyl, Biphenyl oder Phenyl bedeutet, wobei der Phenylrest durch Halogen, Nitro, Phenoxy, Alkyl, Alkoxy oder Halogenalkyl mit jeweils 1 bis 4 Kohlenatomen substituiert sein kann,
Hal Fluor, Chlor oder Brom,
Z CH oder N bedeutet, oder dessen für Pflanzen verträgliches Säureadditions- oder Metallsalz und einen inerten Zusatzstoff.

**5.** Verfahren zur Bekämpfung von Pilzen, dadurch gekennzeichnet, daß man eine fungizid wirksame Menge eines Azolylmethyloxirans der allgemeinen Formel I

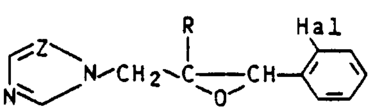 I

in welcher
R 4-Phenylsulfonylphenyl, Naphthyl, Biphenyl oder Phenyl bedeutet, wobei der Phenylrest durch Halogen, Nitro, Phenoxy, Alkyl, Alkoxy oder Halogenalkyl mit jeweils 1 bis 4 Kohlenatomen substituiert sein kann,
Hal Fluor, Chlor oder Brom,
Z CH oder N bedeutet, oder dessen für Pflanzen verträgliches Säureadditions- oder Metallsalz auf die Pilze oder auf durch Pilzbefall bedrohte Materialien, Flächen, Pflanzen oder Saatgüter einwirken läßt.

## Claims

**1.** An azolylmethyloxirane of the formula I

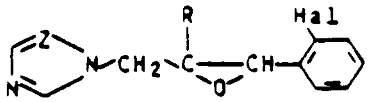

I

where R is 4-phenylsulfonylphenyl, naphthyl, biphenyl or phenyl, and the phenyl radical may be substituted by halogen, nitro or phenoxy or by alkyl, alkoxy or haloalkyl, each of which has 1 to 4 carbon atoms, Hal is fluorine, chlorine or bromine and Z is CH or N, and its plant-tolerated acid addition salts or metal salts.

**2.** A process for the preparation of an azolylmethyloxirane of the formula I as claimed in claim 1, wherein
a) a compound of the formula II

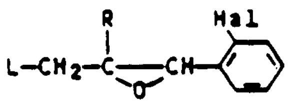

(II)

where R and Hal have the stated meanings and L is a nucleophilically substitutable leaving group, is reacted with a compound of the formula III

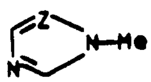

(III)

where Me is a hydrogen atom or a metal atom and Z has the stated meaning, or
b) a compound of the formula II where R and Hal have the above meanings and L is a hydroxyl group, is reacted with a compound of the formula IV

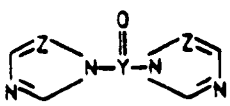

(IV)

where Z has the stated meaning and Y is a carbon or sulfur atom, or
c) a compound of the formula V

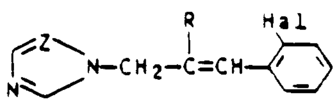

(V)

where Z, R and Hal have the stated meanings, is epoxidized, or
d) a compound of the formula VI

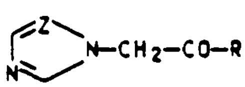

(VI)

where Z and R have the stated meanings, is reacted with a compound of the formula VII

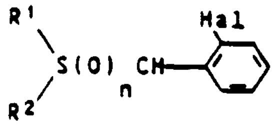

(VII)

where Hal has the stated meaning, $R^1$ and $R^2$ may be identical or different and are each methyl or phenyl, and n is zero or one,
and, if required, the resulting compound is converted to a salt with a physiologically or plant-tolerated acid.

**3.** A fungicidal agent containing a compound of the formula I as claimed in claim 1.

**4.** A fungicidal agent containing an azolylmethyloxirane of the formula I

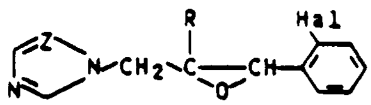

I

where R is 4-phenylsulfonylphenyl, naphthyl, biphenyl or phenyl, and the phenyl radical may be substituted by halogen, nitro or phenoxy or by alkyl, alkoxy or haloalkyl, each of which has 1 to 4 carbon atoms, Hal is fluorine, chlorine or bromine and Z is CH or N, or a plant-tolerated acid addition or metal salt thereof, and an inert additive.

**5.** A method for controlling fungi, wherein a fungicidally effective amount of an azolylmethyloxirane of the formula I

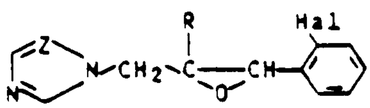

I

where R is 4-phenylsulfonylphenyl, naphthyl, biphenyl or phenyl, and the phenyl radical may be substituted by halogen, nitro or phenoxy or by alkyl, alkoxy or haloalkyl, each of which has 1 to 4 carbon atoms, Hal is fluorine, chlorine or bromine and Z is CH or N, or a plant-tolerated acid addition or metal salt thereof, is allowed to act on the fungi or on materials, areas, plants or seed threatened by fungus attack.

**Revendications**

**1.** Azolylméthyloxirannes de formule générale I

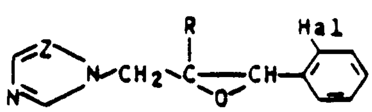

(I)

dans laquelle

R représente un groupe 4-phénylsulfonylphényle, naphtyle, biphényle ou phényle, dans lesquels le noyau phényle peut être substitué par des halogènes, des groupes nitro, phénoxy, alkyle, alcoxy ou halogénoalkyle contenant chacun 1 à 4 atomes de carbone,

Hal représente le fluor, le chlore ou le brome,

Z représente CH ou N, et leurs sels formés par addition avec des acides ou sels métalliques

tolérés par les végétaux.

**2.** Procédé de préparation des azolylméthyloxirannes de formule I de la revendication 1, caractérisé en ce que :

a) on fait réagir un composé de formule II

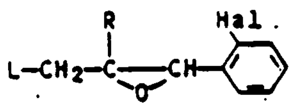

(II)

dans laquelle R et Hal ont les significations indiquées ci-dessus et L représente un groupe éliminable par nucléophilie, avec un composé de formule III

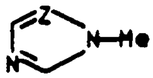

(III)

dans laquelle Me représente un atome d'hydrogène ou un atome métallique et Z a les significations indiquées ci-dessus, ou bien

b) on fait réagir un composé de formule II dans laquelle R et Hal ont les significations indiquées ci-dessus et L représente un groupe hydroxy, avec un composé de formule IV

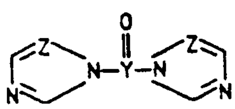

(IV)

dans laquelle Z a les significations indiquées ci-dessus et Y représente un atome de carbone ou de soufre, ou bien

c) on époxyde un composé de formule V

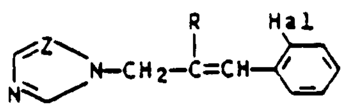

(V)

dans laquelle Z, R et Hal ont les significations indiquées ci-dessus, ou bien

d) on fait réagir un composé de formule VI

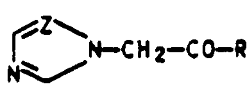

(VI)

dans laquelle Z et R ont les significations indiquées ci-dessus, avec un composé de formule générale VII

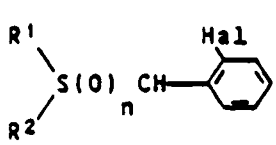

(VII)

dans laquelle Hal a les significations indiquées ci-dessus, $R^1$ et $R^2$, qui peuvent avoir des significations identiques ou différentes, représentent chacun un groupe méthyle ou phényle et n est égal à 0 ou 1, et, le cas échéant, on convertit les composés ainsi obtenus en leurs sels d'acides tolérés par l'organisme et par les végétaux.

**3.** Produit fongicide contenant un composé de formule I de la revendication 1.

**4.** Produit fongicide contenant un azolylméthyloxiranne de formule générale I

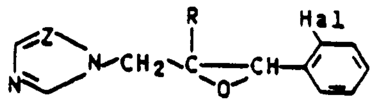

I

dans laquelle

R représente un groupe 4-phénylsulfonylphényle, naphtyle, biphényle ou phényle dans lesquels le noyau phényle peut être substitué par des halogènes, des groupes nitro, phénoxy, alkyle, alcoxy ou halogénoalkyle contenant chacun 1 à 4 atomes de carbone,
Hal représente le fluor, le chlore ou le brome,
Z représente CH ou N, ou l'un de ses sels formés par addition avec un acide ou sels métalliques tolérés par les végétaux, et un additif inerte.

**5.** Procédé pour combattre les mycètes, caractérisé en ce que l'on fait agir sur les mycètes ou sur les matières, surfaces, végétaux ou semences menacés d'une attaque par les mycètes, une quantité fongicide efficace d'un azolylméthyloxiranne de formule générale I

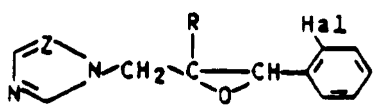

I

dans laquelle

R représente un groupe 4-phénylsulfonylphényle, naphtyle, biphényle ou phényle dans lesquels le noyau phényle peut être substitué par des halogènes, des groupes nitro, phénoxy, alkyle, alcoxy ou halogénoalkyle contenant chacun 1 à 4 atomes de carbone,
Hal représente le fluor, le chlore ou le brome, et
Z représente CH ou N.